# EUROPEAN PATENT SPECIFICATION

(11) **EP 0 826 351 B1**
(45) Date of publication and mention of the grant of the patent: **30.08.2006**
(21) Application number: 97305895.1
(22) Date of filing: 04.08.1997
(51) Int. Cl.: A61F 13/15

(54) **Foldable disposable diaper**
Faltbare Wegwerfwindel
Couche cullotte pliable

(30) Priority: 08.08.1996 JP 20966396
(43) Date of publication of application: 04.03.1998
(73) Proprietor: UNI-CHARM CORPORATION, Shikokuchuo-shi, Ehime-ken (JP)
(72) Inventor: Fujioka, Yoshihisa, Mitoyo-gun, Kagawa-ken (JP); Yamaki, Rumi, Kawanoe-shi, Ehime-ken (JP); Ono, Yoshio, Kawanoe-shi, Ehime-ken (JP)
(74) Representative: Parry, Christopher Stephen

(56) References cited:
- EP-A- 0 336 826
- US-A- 3 963 029
- US-A- 4 557 777
- US-A- 4 685 915
- US-A- 4 690 719
- US-A- 5 536 350

## Description

The present invention relates to a disposable diaper, foldable at a compact size and therefore packable in a package; more specifically, the present invention relates to a compact disposable diaper, which is foldable at a thin thickness and is therefore very convenient for shipment and handy for consumers.

Disposable diaper is composed of a back sheet and a top sheet, along with an absorption core interposed between the two sheets. The back sheet facing outwardly when such diapers are worn comprises a resin sheet, liquid non-permeable but air permeable, while the top sheet facing toward the skin of a wearer is so directly in contact to skin that the top sheet comprises a non-woven fabric or a porous resin sheet, liquid permeable. Alternatively, the absorption core absorbs urine passing through the top sheet and comprises a crushed pulp or a mixture of crushed pulp and super absorbent polymers.

This diaper is prepared by pressing the absorption core to a uniform thickness and thereafter cutting the core into a given shape such as sandglass-like form to interpose the resulting core between the top sheet and the back sheet prior to sealing. The back sheet and the top sheet are of a shape almost similar to the shape of the absorption core, but with a larger outline than the outline of the absorption core. By coating for example a hot-melt adhesive on the back sheet and the top sheet, the two sheets are bonded together at a portion where the absorption core does not exist.

This disposable diaper comprises a front waist region facing the abdominal area of a wearer, a crotch region to be applied to the crotch thereof, and a back waist region facing the dorsal area (back area) thereof. The back sheet and the top sheet protrude at both the right and left ends of each of the front waist region and the back waist region in the width direction of the diapers, where front flaps and back flaps are formed.

Retaining fasteners are fixed on the ends of the back flaps in the width direction, and retaining fasteners are also fixed on the front surface of the back sheet in the front waist region.

When this diaper is worn, the back flaps are pulled out from the dorsal area (back area) of a wearer to the abdominal area along the body outline of the wearer, to be overlaid in the front waist region. Then, the retaining fasteners in the front waist region and the retaining fasteners in the back waist region are attached together.

After production at a plant, such diapers are folded at a small size to be packed in a package for shipping as a manufacturing product.

In such case, both the sides of each of the front waist region and the back waist region are folded over the side of the top sheet along the width direction of the diapers, and then, the front waist region and the back region are overlaid onto the crotch region in three layers.

For folding both the sides of the front waist region in the width direction, generally, the sides are folded at the part where the absorption core is present. Hence, the absorption core is overlaid at least in five layers when the diaper is folded in the three layers. Thus, the dimensional height of the folded diapers is so increased that the dimensional height of the whole package containing a plurality of such diapers overlaid together is also increased.

US-A-4685915 discloses a foldable disposable diaper comprising a back sheet, a liquid permeable top sheet and an absorption core interposed between the two sheets, having a front waist region facing the abdominal area of a wearer, a crotch region to be applied to the crotch thereof, and a back waist region facing the dorsal area thereof, wherein the absorption core is formed in both the front waist region and the back waist region with thin thickness parts having a thickness thinner than the thickness of the absorption core in the crotch region; wherein the front waist region and the back waist region are folded at the thin thickness parts; and wherein the dimensions of the front waist region and the back waist region in the width direction of the diaper are larger than the dimension of the crotch region in the width direction; provided that the direction of aligning the front waist region, the crotch region and the back waist region is defined as the longitudinal direction of the diaper while the direction perpendicular to the longitudinal direction of the diaper is defined as the width direction.

US-A-4690719 comprises an absorbent pad for a diaper of triple thickness in the crotch region and of single thickness in the lateral parts of the waist regions. The pad is formed from a rectangular piece of material comprising incisions in the waist regions and subjected to a double folding around longitudinal lines connecting between them the incisions of the two end zones.

It is an object of the present invention to provide a foldable disposable diaper of a more compact size than those of conventional diapers because the diaper can be folded to a thin thickness.

It is the other object of the present invention to prepare a foldable disposable diaper which can be folded to a thin thickness with no loss of the absorption potency thereof.

The present invention provides a foldable disposable diaper comprising a back sheet, a liquid permeable top sheet and an absorption core interposed between the two sheets, having a front waist region facing the abdominal area of a wearer, a crotch region to be applied to the crotch thereof, and a back waist region facing the dorsal area thereof, wherein the absorption core is formed in both the front waist region and the back waist region with thin thickness parts having a thickness thinner than the thickness of the absorption core in the crotch region, the thickness of the absorption core being uniform excluding the thin thickness parts; wherein the front waist region and the back waist region are folded at the thin thickness parts; and wherein the dimensions of the front waist region and the back waist region in the width direction of the diaper are larger than the dimension of the crotch region in the width direction; provided that the direction of aligning the front waist region, the crotch region and the back waist region is defined as the longitudinal direction of the diaper while the direction perpendicular to the longitudinal direction of the diaper is defined as the width direction; characterised in that each of the waist regions of the absorbent core is formed having a thick thickness central part and laterally outer parts on either side of the thick thickness central part, wherein the laterally outer parts form the thin thickness parts having a thickness thinner than the thickness of the absorption core in the crotch region, and wherein the thickness of the thick thickness central parts of the waist regions is equal to the thickness of the absorption core in the crotch region; wherein the diaper, when in its folded position, is folded along two spaced parallel folding lines (L) each extending in the longitudinal direction of the diaper and dividing the thin thickness parts on one side of the absorption core; the width dimension of the thus divided thin thickness parts positioned inwardly of the respective folding line being the same or greater than the width dimension of the thin thickness parts positioned outwardly of the folding line so that each of the thin thickness parts is folded over onto itself but is not folded over onto the associated thick thickness central part.

Additionally, the thickness of the diaper at the thin thickness part of the absorption core is preferably 1/2-fold or less the thickness of the diaper at the remaining parts excluding the thin thickness part of the absorption core.

The foldable disposable diaper of the present invention is disposable for use for infants, toddlers or aged people, wherein the thickness of the absorption core is thin in the front waist region and the back waist region to prepare thin thickness parts in the absorption core. The disposable diaper is wholly folded in two or three or a plurality, and a given number of such diapers are packed in a package for shipment as a manufacturing product. So as to make the manufacturing product in its entirety at a compact size, thin thickness parts are formed in the absorption core in the front waist region and the back waist region of the foldable disposable diaper of the present invention. When the diaper is folded, the front waist region and the back waist region are folded at the thin thickness parts so these regions are overlaid together at the thin thickness parts. Accordingly, the thickness of the folded and overlaid portion of the diaper can be made so thin that the resulting thickness in the folded diaper can be made thinner than the thickness of conventional diapers with no formation of such thin thickness part. Because the absorption core is still present at the thin thickness parts, the area of the absorption core in the whole diaper is never reduced. Because the thickness of the folded diaper can be made thin, additionally, the absorption core existing in the crotch region of the diaper and to be applied to the crotch of a wearer can be thicker than conventional diapers.

An embodiment of the invention is described below with reference to the accompanying drawings, in which:
Fig.1(A) is a developed plane view of the foldable disposable diaper of the present invention, which is viewed from the side toward skin;
Fig.1(B) is a plane view of the diaper of Fig.1(A), which is folded inwardly along the line L-L;
Fig.1(C) is a perspective view depicting the diaper of Fig.1(B), which is folded inwardly along the line M-M;
Fig.2 (A) is a cross sectional view of the diaper of Fig.1(A) along the line IIA-IIA;
Fig.2 (B) is a cross sectional view of the diaper of Fig.1(B) along the line IIB-IIB;
Fig.2 (C) is a cross sectional view of the diaper of Fig.1(C) along the line IIC-IIC; and
Fig.3 is a front view of the diaper at a state when the diaper is actually worn.

The present invention will now be described with reference to drawings.

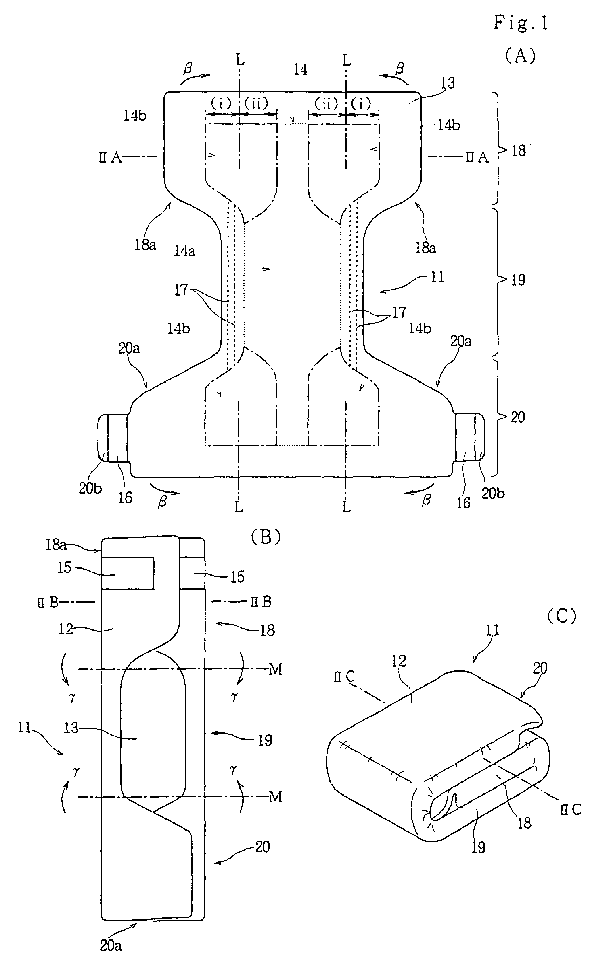

As shown in Fig.1(A), diaper 11 is in a lamination structure where absorption core 14 is interposed between back sheet 12 and top sheet 13. The back sheet 12 is faced outwardly when the diaper 11 is worn, and comprises a resin sheet, liquid non-permeable but air permeable, so as not to ooze water such as urine outside. Alternatively, the top sheet 13 facing the skin side of a wearer is directly in contact to the skin of the wearer, and the top sheet 13 therefore comprises a non-woven fabric or a porous resin sheet, liquid permeable, which can permeate spotted urine into the absorption core. Additionally, the absorption core 14 absorbs urine permeating through the top sheet 13, and comprises a highly absorbent crushed pulp, or a mixture of a crushed pulp and a super absorbent polymer.

As shown in Fig.1(A), the diaper 11 is formed in a sandglass-like form. The narrow middle region of the sandglass-like form is the crotch region 19, while the front and back parts thereof in wider forms are individually the front waist region 18 and the back waist region 20.

The absorption core 14 in the sandglass-like form is arranged from the crotch region 19 to the front waist region 18 and the back waist region 20. In the front waist region 18 and the back waist region 20, the absorption core 14 is prepared, in the regions surrounded with an alternate long and short dash line in both the left and right regions of the diaper, as thin thickness part 14b of a thickness thinner than the thickness of the absorption core in the remaining region. In the present example, the thickness of the absorption core 14 is uniform in the region excluding the thin thickness part 14b, and so as to discriminate the region from the thin thickness part 14b, the region is designated as thick thickness part 14a.

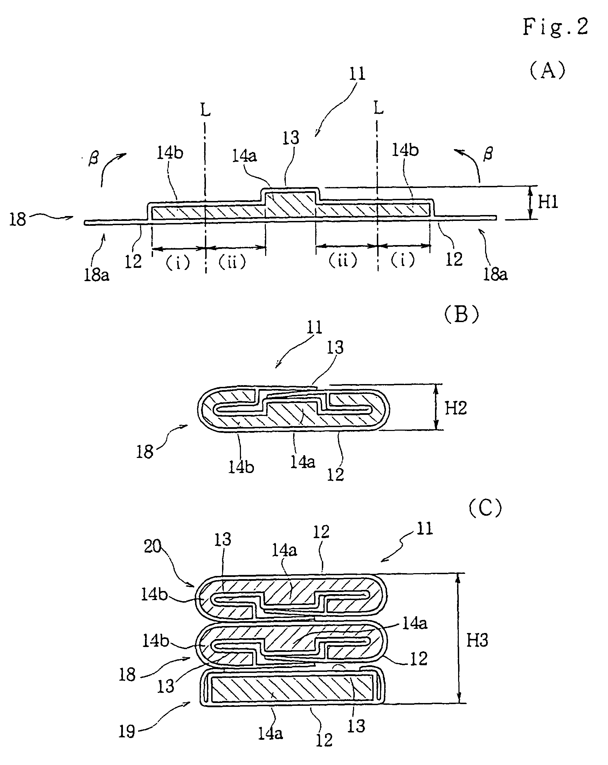

As shown in the cross sectional view of Fig.2(A), the thickness of the diaper 11 at the thin thickness part 14b is 1/2×H1 or less, provided that the thickness of the diaper at the thick thickness part 14a is defined as H1.

The back sheet 12 and the top sheet 13 are in a sandglass-like form, nearly similar to the form of the absorption core 14, and these sheets are wholly larger individually than the absorption core 14. The absorption core 14 is put in the center between the back sheet 12 and the top sheet 13, to be interposed between the back sheet 12 and the top sheet 13. At parts where the absorption core 14 is not present between the back sheet 12 and the top sheet 13, namely at the margins in the width direction and the longitudinal direction, a hot-melt adhesive or the like is coated on the margins where the back sheet 12 and the top sheet 13 face to each other, to attach the back sheet 12 and the top sheet 13 together. In such manner, the absorption core 14 is sealed in between the back sheet 12 and the top sheet 13. The back sheet 12 and the top sheet 13 protrude in the front waist region 18 and the back waist region 20 of the diaper 11 in the width direction of the diaper 11. The protruding parts form individually front flap 18a, 18a and back flap 20a, 20a.

At the end parts of the crotch region 19 in the width direction of the diaper 11 are arranged elastic members 17 elongating in the longitudinal direction of the diaper. At an elongated state between the back sheet 12 and the top sheet 13, the elastic members 17 comprising for example flat elastic braid adhere to the back sheet 12 and the top sheet 13 by means of a hot-melt adhesive. The elastic members can form gathering. When the diaper 11 is worn, the gathering facing the thigh part of a wearer elastically presses the thigh part.

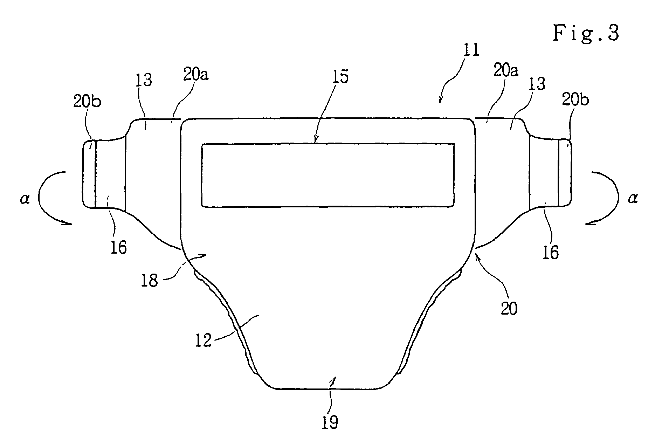

Herein, the back waist region 20 of the diaper 11 is formed wider than the front waist region 18, and protrusion 20b is formed on each end of the back waist region 20 in the width direction. Retaining fastener 16 is fixed on the protrusion 20b, and the protrusion 20b is generally at a folded state on the inner side of the diaper 11, excluding when the diaper 11 is worn. When the diaper 11 is worn, the back waist region 20 is applied to the back of a wearer while the crotch region 19 is applied to the crotch thereof. Then, the diaper is folded and flapped in front to apply the front waist region 18 abdominally, at such a state as shown in Fig.3. Subsequently, the back flaps 20a, 20a are drawn forward along the body outline of a wearer toward the abdominal area, and thereafter, each retaining fastener 16 of each protrusion 20b is fastened on the retaining fastener 15 fixed on the back sheet 12 in the front waist region 18. The diaper 11 is worn in such fashion.

Alternatively, instead of the retaining fastener 15, film is fixed on the surface of the back sheet 12 in the front waist region 18; instead of the retaining fastener 16, an adhesive tape is fixed in the back waist region 20. In this case, the adhesive tape in the back waist region 20 adheres to the film in the front waist region 18, to attach the front waist region 18 and the back waist region 20 together.

After the production of the disposable diaper 11, the front flaps 18a, 18a on both the right and left sides in the front waist region 18 as well as the back flaps 20a, 20a on both the sides in the back waist region 20 are folded along the line L-L parallel to the longitudinal direction, toward the direction shown by arrow *β*, so that the diaper 11 is folded as shown in Fig.1(B). Furthermore, the front waist region 18 and the back waist region 20 are folded along the line M-M parallel to the width direction of the diaper toward the direction shown by arrow *γ* to be overlaid on the crotch region 19, so that the diaper is folded at such a state as shown in Fig.1(C). The diaper folded at the state shown in Fig.1(C) is packed in a package and the like.

So as to make the thickness of the folded diaper 11 as thin as possible, the line L-L should be present in a region where the absorption core 14 is not present in the crotch region 19. Furthermore, the line L-L should be present at the end side in the width direction of the diaper from a parallel line to the longitudinal line of the diaper, the parallel line dividing the thin thickness part 14b of the absorption core in halves on right and left sides and the thin thickness part 14b having been formed individually in the front waist region 18 and the back waist region 20. More specifically, when the diaper is divided along the line L-L as shown in Fig.1(A) and Fig. 2 (A), the dimensional width (i) of the thin thickness part on the end side in the width direction of the diaper 11 is the same as or smaller than the dimensional width (ii) of the thin thickness part on the central side. Accordingly, the diaper 11 should necessarily be folded along the line L-L as shown in Fig.2(B), so that the thin thickness parts 14b, 14b in the front flaps 18a, 18a formed on the right and left ends of the front waist region 18 might be overlaid together. Similarly, the back flaps 20a, 20a should necessarily be folded so that the thin thickness parts thereof might be overlaid together.

Because the thickness of the diaper 11 at the thin thickness part 14b is 1/2-fold or less the thickness of the diaper at the thick thickness part 14a, the thickness H2 of the thin thickness parts 14b, 14b overlaid together is almost H1 or less.

When the diaper 11 is folded at a state as shown in Fig.1(C) the front waist region 18 and the back waist region 20, both folded along the line L-L as shown in Fig.1(B), are overlaid on the crotch region 19 as shown in the cross sectional view of Fig.2(C). The thickness H2 of the front waist region 18 and the back waist region 20, both overlaid on the crotch region 19, is almost the same as the thickness H1 of the thin thickness part 14a of the diaper, as described above, and therefore, the total thickness H3 of the diaper 11 at a folded state as shown in Fig.1(C) is the thickness as the sum of the thickness H1 of the crotch region 19 and the thickness H2 of the front waist region 18 and the back waist region 20, which is nearly the same thickness as 3×H1.

In accordance with the present invention, therefore, the thickness of the folded diaper can be suppressed to about 3-fold the thickness of the diaper at the developed state. The thickness of conventional diapers at their folded state is 5-fold or more the thickness of the diapers at their developed state. When the thickness of the thick thickness part 14a of the diaper 11 is the same thickness of conventional diapers, therefore, the diaper of the present invention can be folded at a thickness 3/5-fold the thickness of the conventional diapers. When the inventive diaper is packed in a package for shipment as a product, accordingly, the bulkiness of the product can be made less, so that the product can be compact. Hence, a large number of such products can be shipped at one time, compared with conventional diapers, and therefore, the product is also handy for consumers.

Because the thickness of the absorption core in the crotch region 19 is not thin, the absorption potency of the diaper is never reduced. Even when the thickness of the absorption core in the crotch region 19 is thicker than the thickness of conventional diapers, on contact, the diaper can be made thin compared with conventional diapers.

The present invention has been described insofar in one example as the open-type disposable diaper. However, the present invention is not limited to the example. In accordance with the present invention, a brief-type diaper product can be made compact, by forming such thin thickness parts in the front waist region and the back waist region, whereby the thickness of the folded diaper can be made thin.

In accordance with the present invention, as has been described hereinabove, the thin thickness parts are formed in the front waist region and the back waist region of a disposable diaper to make the thickness of the folded diaper thin with no reduction of the thickness of the absorption core in the crotch region or with no loss of the absorption potency of the diaper. Thus, the diaper can be made thinner.

When the diaper of the present invention is folded and packed in a package for shipment as a product, the product can be made more compact. Compared with conventional diapers, a greater number of such products can be shipped at one time, which can contribute to the reduction of shipment cost. Because the product is not bulky, additionally, the product is handy for purchasers and consumers.

When the diaper is to be folded inwardly along a line parallel to the longitudinal direction of the diaper, furthermore, the line should be formed on each of the marginal sides in the width direction of the diaper, from the line parallel to the longitudinal line of the diaper, the parallel line dividing the thin thickness parts individually formed in the front waist region and the back waist region in halves on right and left sides, so that the area of the thin thickness parts in the inner region of the diaper, on which the end parts of the front waist region and the back waist region are overlaid, can be made larger than the area of the thin thickness parts in the front waist region and the back waist region to be folded inwardly. Hence, the thick parts of the diaper are never overlaid together to make the thickness of the folded diaper thinner.

## Claims

1. A foldable disposable diaper comprising a back sheet (12), a liquid permeable top sheet (13) and an absorption core (14) interposed between the two sheets, having a front waist region (18) facing the abdominal area of a wearer, a crotch region (19) to be applied to the crotch thereof, and a back waist region (20) facing the dorsal area thereof, wherein the absorption core (19) is formed in both the front waist region and the back waist region with thin thickness parts (14b) having a thickness thinner than the thickness of the absorption core in the crotch region (19), the thickness of the absorption core being uniform excluding the thin thickness parts (14b); wherein the front waist region and the back waist region are folded at the thin thickness parts; and
wherein the dimensions of the front waist region (18) and the back waist region (20) in the width direction of the diaper are larger than the dimension of the crotch region (19) in the width direction; provided that the direction of aligning the front waist region, the crotch region and the back waist region is defined as the longitudinal direction of the diaper while the direction perpendicular to the longitudinal direction of the diaper is defined as the width direction; **characterised in that** each of the waist regions (18, 20) of the absorbent core is formed having a thick thickness central part (14A) and laterally outer parts on either side of the thick thickness central part, wherein the laterally outer parts form the thin thickness parts (14b) having a thickness thinner than the thickness of the absorption core in the crotch region (19), and wherein the thickness of the thick thickness central parts of the waist regions is equal to the thickness of the absorption core in the crotch region;
wherein the diaper, when in its folded position, is folded along two spaced parallel folding lines (L) each extending in the longitudinal direction of the diaper and dividing the thin thickness parts (14b) on one side of the absorption core; the width dimension of the thus divided thin thickness parts positioned inwardly of the respective folding line being the same or greater than the width dimension of the thin thickness parts positioned outwardly of the folding line so that each of the thin thickness parts (14b) is folded over onto itself but is not folded over onto the associated thick thickness central part.

2. A foldable disposable diaper according to claim 1, wherein the folding lines over which the thin thickness parts (14b) are folded are positioned so as not to overlay the absorption core in the crotch region.

3. A foldable disposable diaper according to claim 2, wherein the front waist region and back waist region are further folded along two lines each parallel to the width direction of the diaper so that the front waist region and the back waist region with both their ends having already been folded might be folded in the width direction to be overlaid over the crotch region.

4. A foldable disposable diaper according to any preceding claim, wherein the thickness of the diaper at the thin thickness part (s) of the absorption core is 1/2-fold or less the thickness of the diaper excluding the thin thickness part (s).

## Patentansprüche

1. Zusammenfaltbare Wegwerfwindel mit einer rückwärtigen Lage (12), einer flüssigkeitsdurchlässigen oberen Lage (13) und einem zwischen den beiden Lagen angeordneten absorbierenden Kern (14), die einen dem Bauchbereich eines Trägers zugewandten vorderen Taillenbereich (18), einen Schrittbereich (19) für seinen Schritt und einen seiner Rückenfläche zugewandten hinteren Taillenbereich (20) aufweist, wobei der absorbierende Kern (14) sowohl im vorderen als auch im hinteren Taillenbereich mit Dünnteilen (14b) ausgebildet ist, deren Dicke geringer als die des absorbierenden Kerns im Schrittbereich (19) ist, wobei die Dicke des absorbierenden Kerns mit Ausnahme der Dünnteile (14b) gleichförmig ist und wobei der vordere und der hintere Taillenbereich an den Dünnteilen gefaltet sind, und
wobei die Maße des vorderen Taillenbereichs (18) und des hinteren Taillenbereichs (20) in Breitenrichtung der Windel größer als das Maß des Schrittbereichs (19) in Breitenrichtung ist, wobei die Richtung, in der der vordere Taillenbereich, der Schrittbereich und der hintere Taillenbereich aufgereiht sind, als Längsrichtung der Windel und die zur Längsrichtung senkrechte Richtung als Breitenrichtung definiert sind,
**dadurch gekennzeichnet, dass** die Taillenbereiche (18, 20) des absorbierenden Kerns jeweils mit einem mittleren Dickteil (14a) und auf dessen beiden Seiten mit Queraußenteilen ausgebildet sind, von denen die Queraußenteile die Dünnteile (14b) mit geringerer Dicke als der des absorbierenden Kerns im Schrittbereich (19) bilden, und die Dicke der mittleren Dickteile der Taillenbereiche gleich der Dicke des absorbierenden Kerns im Schrittbereich ist,
wobei die Windel in ihrer gefalteten Stellung entlang zweier paralleler Faltlinien (L) gefaltet ist, die mit Abstand zueinander jeweils in Längsrichtung der Windel verlaufen und die Dünnteile (14b) jeweils auf einer Seite des absorbierenden Kerns teilen, wobei das Breitenmaß der so geteilten Dünnteile einwärts der entsprechenden Faltlinie gleich oder größer als das Breitenmaß der Dünnteile auswärts der Faltlinie ist, so dass die Dünnteile (14b) jeweils auf sich selbst und nicht auf das zugeordnete mittlere Dickteil hinübergefaltet sind.

2. Windel nach Anspruch 1, wobei die Faltlinien, über die die Dünnteile (14b) gefaltet sind, so angeordnet sind, dass sie im Schrittbereich nicht über dem absorbierenden Kern liegen.

3. Windel nach Anspruch 2, wobei der vordere und der hintere Taillenbereich außerdem entlang zweier jeweils zur Breitenrichtung der Windel paralleler Linien gefaltet sind, so dass der vordere Taillenbereich und der hintere Taillenbereich, deren beide Enden jeweils bereits gefaltet sind, bezüglich der Breitenrichtung über den Schrittbereich liegend gefaltet werden können.

4. Windel nach einem der vorhergehenden Ansprüche, wobei die Dicke der Windel am Dünnteil bzw. den Dünnteilen des absorbierenden Kerns die Hälfte oder weniger der Dicke der Windel ausgenommen des bzw. der Dünnteile ist.

## Revendications

1. Couche-culotte jetable pliable comprenant une feuille inférieure (12), une feuille supérieure perméable aux liquides (13) et une partie centrale d'absorption (14) interposée entre les deux feuilles, comportant une région de taille avant (18) face à la zone abdominale d'un porteur, une région d'entre-jambes (19) devant être appliquée à l'entre-jambes de celui-ci, et une région de taille arrière (20) face à la zone dorsale de celui-ci, dans laquelle la partie centrale d'absorption (19) est formée dans à la fois la région de taille avant et la région de taille arrière, avec des parties à épaisseur mince (14b) qui présentent une épaisseur plus mince que l'épaisseur de la partie centrale d'absorption dans la région d'entre-jambes (19), l'épaisseur de la partie centrale d'absorption étant uniforme, à l'exception des parties à épaisseur mince (14b), dans laquelle la région de taille avant et la région de taille arrière sont pliées au niveau des parties à épaisseur mince, et
dans laquelle les dimensions de la région de taille avant (18) et de la région de taille arrière (20) dans le sens de la largeur de la couche-culotte sont plus importantes que les dimensions de la région d'entre-jambes (19) dans le sens de la largeur, à condition que le sens d'alignement de la région de taille avant, de la région d'entre-jambes et de la région de taille arrière soit défini en tant que sens de la longueur de la couche-culotte alors que le sens perpendiculaire au sens de la longueur de la couche-culotte est défini en tant que sens de la largeur, **caractérisée en ce que** chacune des régions de taille (18, 20) de la partie centrale absorbante est formée en présentant une partie centrale à épaisseur importante (14A), et latéralement des parties extérieures sur chaque côté de la partie centrale à épaisseur importante, où les parties latéralement extérieures forment les parties à épaisseur mince (14b) présentant une épaisseur plus fine que l'épaisseur de la partie centrale d'absorption dans la région d'entre-jambes (19), et où l'épaisseur des parties centrales à épaisseur importante des régions de taille est égale à l'épaisseur de la partie centrale d'absorption dans la région d'entre-jambes,
dans laquelle, lorsqu'elle se trouve dans sa position pliée, est pliée le long de deux lignes de pliage parallèles espacées (L) s'étendant chacune dans le sens de la longueur de la couche-culotte, et séparant les parties à épaisseur mince (14b) sur un côté de la partie centrale d'absorption, les dimensions en largeur des parties à épaisseur mince ainsi divisées, positionnées vers l'intérieur de la ligne de pliage respective, étant identiques aux ou plus importantes que les dimensions en largeur des parties à épaisseur mince positionnées vers l'extérieur de la ligne de pliage, de sorte que chacune des parties à épaisseur mince (14b) est pliée sur elle-même, mais n'est pas pliée sur la partie centrale à épaisseur importante associée.

2. Couche-culotte jetable pliable selon la revendication 1, dans laquelle les lignes de pliage sur lesquelles les parties à épaisseur mince (14b) sont pliées, sont positionnées de façon à ne pas recouvrir la partie centrale d'absorption dans la région d'entrejambes.

3. Couche-culotte jetable pliable selon la revendication 2, dans laquelle la région de taille avant et la région de taille arrière sont en outre pliées le long de deux lignes, chacune étant parallèle au sens de la largeur de la couche-culotte, de sorte que la région toutes les deux leurs extrémités déjà pliées, peuvent être pliées dans le sens de la largeur afin de recouvrir la région d'entre-jambes;

4. Couche-culotte jetable pliable selon l'une quelconque des revendications précédentes, dans laquelle l'épaisseur de la couche-culotte au niveau de la ou des parties à épaisseur mince de la partie centrale d'absorption représente, une fois pliée en deux, l'épaisseur ou moins de la couche-culotte en excluant la ou les parties à épaisseur mince.
